# EUROPÄISCHE PATENTSCHRIFT

(11) **EP 3 708 101 B1**
(45) Veröffentlichungstag und Bekanntmachung des Hinweises auf die Patenterteilung: **27.08.2025**
(21) Anmeldenummer: 20171105.8
(22) Anmeldetag: 08.09.2014
(51) Int. Cl.: A61B 18/12, A61B 18/14, A61B 18/16

(54) **SYSTEM ZUR GLEICHZEITIGEN GEWEBEKOAGULATION UND GEWEBEDISSEKTION**
SYSTEM FOR SIMULTANEOUS TISSUE COAGULATION AND TISSUE DISSECTION
SYSTÈME DE COAGULATION TISSULAIRE ET DE DISSECTION TISSULAIRE SIMULTANÉES

(43) Veröffentlichungstag der Anmeldung: 16.09.2020
(62) Teilanmeldung aus: 14183945.6
(73) Patentinhaber: Erbe Elektromedizin GmbH, 72072 Tübingen (DE)
(72) Erfinder: SCHALL, Heiko, 72622 Nürtingen (DE); BRODBECK, Achim, 72555 Metzingen (DE); WEILER, Rolf, 72074 Tübingen (DE); MAYER, Volker, 72072 Tübingen (DE); AMANN, Tobias, 72351 Geislingen (DE)
(74) Vertreter: Rüger Abel Patentanwälte PartGmbB

(56) Entgegenhaltungen:
- WO-A1-00/47124
- WO-A1-94/26228
- US-A1- 2008 172 052
- US-A1- 2010 137 854
- US-A1- 2011 245 826
- US-B2- 7 803 156
- US-B2- 8 394 094

## Beschreibung

Es sind chirurgische Instrumente bekannt, mit denen das Schneiden von Gewebe (Dissektion) und die Versiegelung von Gewebe (Koagulation) durchführbar sind.

Aus der US 8,394,094 B2 und US2010137854A1 ist ein solches Instrument bekannt. Es ist nach Art einer Zange ausgebildet, zwischen deren beiden Branchen Gewebe zu fassen ist. An einer der Branchen sind sowohl Koagulationselektroden als auch eine Schneidelektrode angebracht. Die andere Branche dient als Gegenelektrode. Außerdem ist ein der Schneidelektrode gegenüber liegendes, beweglich gehaltenes Widerlager vorgesehen. Die Schneidelektrode ist in einem Kunststoffteil gefasst, aus dem es mit seiner als Schneidkante dienenden Stirnfläche und sich daran anschließenden schmalen Flankenbereichen herausragt.

Die WO 00/47124 offenbart ein ähnliches Instrument mit beweglich gelagerter Schneidelektrode. Die Schneidelektrode ist zwischen zwei Versiegelungselektroden gehalten, denen in Nuten eingetieft angeordnete Gegenelektroden zugeordnet sind. Zur Versorgung dieses Instruments dient ein elektrischer Generator, aus dessen Ausgangsspannung über eine Transformatoranordnung sowohl die Koagulationsspannung als auch die Schneidspannung abgeleitet werden. Der Transformator weist eine ein- oder zweiteilige Eingangswicklung und zwei Ausgangswicklungen auf. In einer abgewandelten Ausführungsform sind die beiden Ausgangswicklungen an einer Stelle miteinander verbunden. Jedenfalls aber ist eine galvanische Trennung zwischen Eingangswicklung und Ausgangswicklung vorhanden. Damit muss von dem Kern des Transformators sowohl die zum Koagulieren wie auch die zum Schneiden benötigte Leistung übertragen werden.

Versuche mit verschiedenen Instrumentenkonfigurationen haben gezeigt, dass bei gleichzeitiger Durchführung von Versiegelung und Schnitt eine komplizierte Abhängigkeit zwischen dem chirurgischen Ergebnis und technischen Parametern besteht. Sowohl die räumliche Anordnung der Koagulations- und Schneidelektroden wie auch die Art der Bereitstellung elektrischer Leistung bzw. Energie haben einen erheblichen Einfluss auf die Qualität der Versiegelung und des Schnitts. Beide Einflussfaktoren wirken auf die Stromverteilung und die Verteilung mechanischer Kräfte auf das Gewebe und somit auf das an dem biologischen Gewebe erzielte Ergebnis.

Es ist Aufgabe der Erfindung, ein System bereitzustellen, welches die sichere und schnelle Gewebetrennung und Versiegelung mittels elektrischer Energie gestattet.

Diese Aufgabe wird von dem System nach Anspruch 1 gelöst:

Das erfindungsgemäße System umfasst ein Instrument zur Behandlung von biologischem Gewebe sowie eine zugehörige Energieversorgungsanordnung. Das Instrument und die Energieversorgungsanordnung sind auf besondere Weise aufeinander abgestimmt. Das Instrument ist darauf eingerichtet, gleichzeitig an räumlich voneinander beabstandeten Stellen Gewebe zu schneiden und zu koagulieren bzw. zu versiegeln. Dazu sind eine Schneidelektrode, eine Koagulationselektrode bzw. Versiegelungselektrode und eine Gegenelektrode vorgesehen. Die Schneidelektrode und die Koagulationselektrode sind voneinander beabstandet und vorzugsweise durch einen Gewebeaufnahmeraum getrennt. Die Branchen des Instruments, die die Elektroden aufweisen, können durch entsprechende Bedienhebel beispielsweise von Hand bewegt, geschlossen und geöffnet werden. Die Erfindung ist aber nicht auf solche Instrumente beschränkt, sie kann auch bei motorisch gesteuerten Branchen wie beispielsweise bei robotergesteuerten Branchen Anwendung finden. Die Energieversorgunganordnung enthält einen Transformator, der zwei Ausgänge aufweist, einen, der mit der Schneidelektrode verbunden ist und einen, der mit der Koagulationselektrode verbunden ist. Die Ausgänge sind über mindestens ein Strombegrenzungselement in Gestalt eines Koppelkondensators voneinander entkoppelt. Das Strombegrenzungselement beschränkt den Stromfluss zu der Schneidelektrode und/oder zu der Koagulationselektrode, so dass ein an der Schneidelektrode eventuell vorhandener niedriger Gewebewiderstand das Koagulationsergebnis nicht beeinträchtigt und ein von der Schneidelektrode ausgehender Stromfluss nicht zu unerwünschten Koagulationserscheinungen führt. Umgekehrt beeinträchtigt ein an der Koagulationselektrode vorhandener niedriger Gewebewiderstand das Schneidergebnis nicht.

Es ist möglich, dass zwischen dem ersten Ausgang und der Schneidelektrode ein Strombegrenzungselement vorgesehen ist. Dies wird als vorteilhaft angesehen. Zusätzlich oder alternativ kann zwischen dem zweiten Ausgang und der Koagulationselektrode ein Strombegrenzungselement vorgesehen sein. Durch entsprechende Auslegung der Strombegrenzungselemente können die Behandlungsströme an der Koagulationselektrode und an der Schneidelektrode unabhängig voneinander eingestellt werden. Zusätzlich kann das Koagulationsergebnis über die an den Ausgängen des Transformators anstehenden Spannungen beeinflusst werden. Über das eine, oder die beiden, Strombegrenzungselemente wird aus Sicht der jeweiligen Elektrode der Innenwiderstand der HF-Quelle wie gewünscht eingestellt, während über die Wicklungen des Transformators die Leerlaufspannungen zumindest an dem mit der Schneidelektrode verbundenen Ausgang wie gewünscht eingestellt wird. Durch separate Einstellung von Spannung und Strom kann die Speisung der Schneidelektrode und der Versiegelungselektrode bzw. Koagulationselektrode optimiert werden.

Erfindungsgemäß ist der Querschnitt beider Branchen, sowohl der ersten wie auch der zweiten Brache, "U-förmig gebildet, wodurch ein Gewebeaufnahmeraum entsteht. Dieser Gewebeaufnahmeraum ist so angeordnet, dass er sich sowohl in die erste und auch in die zweite Branche hinein erstreckt. Bei geschlossenen Branchen kann zwischen diesen ein Spalt, ein Quetschspalt entstehen. In dem Gewebeaufnahmeraum gehaltenes Gewebe hintergreift den Quetschspalt und führt zu einem formschlüssigen Halt des Gewebes in dem Instrument auch wenn die Schneidelektrode das gehaltene Gewebe bereits durchtrennt hat. Die Strombegrenzung durch den Koppelkondensator oder ein sonstiges Strombegrenzungselement in Verbindung mit der Gestaltung (Geometrie, Isolation und Anordnung) des Schneidelements verhindert ein Schrumpfen des Gewebesaums beim Schneiden.

Die Ausbildung der Schneidelektrode im Bereich ihrer Stirnseite ist für die Schnittqualität mitentscheidend. Dabei zeichnen sich die leitenden Bereiche in Verbindung mit den isolierenden Bereichen an der Stirnseite der Schneidelektrode für den Stromfluss aus. Erfindungsgemäß ist die Schneidelektrode lediglich an ihrer Schmalseite elektrisch leitend ausgebildet. An ihren Seitenflächen, die dem Quetschspalt zugewandt sind, ist sie weitestgehend elektrisch isolierend ausgebildet. Dadurch wird ein sauberer Schnitt ermöglicht. Außerdem kann erreicht werden, dass die Koagulation des in dem Aufnahmeraum vorhandenen Gewebes geringer ist als die Koagulation des in dem Quetschspalt gehaltenen Gewebes. Auf diese Weise wird die Gewebeschrumpfung in dem Gewebeaufnahmeraum minimiert und der Effekt des Festhaltens des Gewebes während der Koagulation maximiert.

Es kann auch vorteilhaft sein, wenn die Schneidelektrode an ihren Seitenflächen teilweise elektrisch leitend ausgebildet ist. Dabei umfasst dieser Bereich ausgehend von der Stirnseite weniger als 500µm vorzugsweise 300 µm, so dass die Stirnseite mit den Seitenflächen in diesem Bereich aus der Isolation vorsteht. Durch ein Schneidelement welches an seiner Stirnseite schmale leitende Bereiche aufweist, kann eine ausreichende Schnittqualität sichergestellt werden ohne dass dabei das Gewebe im Gewebeaufnahmeraum zu stark schrumpft.

Der Schneidelektrode kann ein elektrisch isolierendes Widerlagerelement zugeordnet sein. Dieses ist beispielsweise aus Kunststoff, beispielsweise aus Elastomer oder beispielweise aus Keramik ausgebildet. Es ist jedoch auch möglich, ein metallisches Widerlager zu verwenden, das entweder mit der Gegenelektrode verbunden oder elektrisch isoliert angebracht ist. Durch die Beweglichkeit des Widerlagerelements kann es sich entsprechend der wechselnden Dicke des zwischen Widerlagerelement und Schneidelektrode gehaltenen Gewebes ausrichten. Vorzugsweise ist das Widerlagerelement federnd gelagert. Es kann somit während des Schneidvorgangs dazu beitragen, den Hauptteil der Klemmkräfte auf den Quetschspalt der Koagulationselektroden zu lenken und gleichzeitig das schrumpfende Gewebe an die Schneidelektrode heranzudrücken, was einerseits den Schneidvorgang und andererseits das Halten des Gewebes im Quetschspalt unterstützt.

Die Auflagefläche des Widerlagerelements kann teilweise oder ganz eben sein. Insbesondere ist das Widerlagerelement in seiner Form vorzugsweise an die Form der Schneidelektrode angepasst, so dass die Schneidelektrode vorzugsweise entlang ihrer gesamten Länge an dem Widerlagerelement Anlage findet. Die Koagulationselektrode kann durch eine Reihe voneinander beabstandeter Einzelelektroden gebildet sein. Gleiches gilt vorzugsweise für die Gegenelektrode. Die einzelnen beabstandeten Einzelelektroden der Koagulationselektrode sind untereinander vorzugsweise elektrisch verbunden. Ebenso sind die Einzelelektroden der Gegenelektrode vorzugsweise untereinander elektrisch verbunden. Die Einzelelektroden der Koagulationselektrode und der Gegenelektrode sind aber zueinander vorzugsweise nicht überlappend ausgerichtet, so dass bei geschlossenen Branchen selbst wenn kein Gewebe dazwischen ist, kein elektrischer Kurzschluss auftreten kann.

Der Transformator ist vorzugsweise derart ausgebildet, dass der mit der Schneidelektrode verbundene Ausgang eine höhere Spannung liefert als der mit der Koagulationselektrode verbundene Ausgang. Damit wird die Aktion der Schneidelektrode und der Gegenelektrode nicht nur durch die Elektrodenform, sondern zugleich durch die Speisung der Elektroden bestimmt.

Vorzugsweise ist die Impedanz des Strombegrenzungselements größer als der Innenwiderstand des Transformators an seinem ersten bzw. an seinem zweiten Ausgang. Die Impedanz ist dabei auf die genutzte Hochfrequenz bezogen. Damit begrenzt das Strombegrenzungselement (z.B. der Koppelkondensator) den Strom, so dass selbst bei Kurzschluss an den angeschlossenen Elektroden die Spannung an der jeweils anderen Elektrode nicht zusammenbricht.

Der Transformator wird vorzugsweise mit der Koagulationsspannung versorgt. Weiter vorzugsweise ist der Transformator als Spartransformator ausgebildet. Damit muss der Kern des Transformators lediglich die an einem seiner Ausgänge abzugebende Leistung übertragen, die an dem anderen Ausgang abgegebene Leistung ist nicht über den Transformatorkern geführt. Außerdem wird die Anzahl der Wicklungen reduziert, da eine Wicklung von der Primär- und Sekundärseite gemeinsam genutzt wird. Dies ermöglicht den Aufbau besonders platz- und gewichtssparender Transformatoren, die auch im Instrument untergebracht werden können. Alternativ kann der Transformator direkt mit der Schwingspule des Generators magnetisch gekoppelt sein, wobei die Ausgänge des Transformators über mindestens ein geeignetes Strombegrenzungselement (z.B. Koppelkondensator, ohmscher Widerstand, RC-Kombination) mit Ausgängen des Generators verbunden sind.

Die oben beschriebene erfindungsgemäße Auslegung der Energieversorgungseinrichtung mit der oben beschriebenen erfindungsgemäßen Auslegung des Instruments erlaubt die Behandlung von Gewebe, wobei der Trennprozess des Gewebes, das Schneiden des Gewebes, beendet ist, bevor der Versiegelungsprozess des Gewebes beendet ist und trotzdem das Bluten von getrenntem Gewebe vermieden bzw. nahezu vermieden wird. Dieses überraschende Ergebnis, dass qualitativ hochwertige Gewebetrennungen durchgeführt werden können, obwohl der Scheideprozess vor dem Versiegelungsprozess beendet ist, führt dazu, dass die Gesamtdauer des Prozesses von Gewebe versiegeln und Gewebe schneiden reduziert werden kann, was wiederum zu Einsparungen während der Anwendung führt. Beispielsweise sind Gesamtdauern von kleiner drei Sekunden für den Gesamtprozess von Schneiden und versiegeln typisch, davon fallen auf den Gewebeschnitt in der Regel weniger als 0,5 Sekunden. Die restliche Behandlungszeit fällt auf den Prozess der Versiegelung.

Für besondere Anwendungen ist es auch möglich, die Energieversorgungsanordnung so auszulegen, dass das Schneidelement als Versiegelungselektrode wirkt oder als passives Bauteil ohne elektrische Wirkung ausgebildet ist. Dazu kann zwischen dem ersten Ausgang des Transformators und der Schneidelektrode ein Schalter, Potentiometer oder sonstiges elektrisches Bauelement angeordnet sein. Die Branchen des Instruments werden entsprechend den oben beschriebenen Merkmalen ausgelegt. Ein so ausgebildetes System ermöglicht eine qualitativ hochwertige Versiegelung von Gefäßen.

Weitere Einzelheiten vorteilhafter Ausführungsformen der Erfindung sind der Zeichnung, der Beschreibung oder Unteransprüchen zu entnehmen. Es zeigen:
Figur 1 Das System, in schematisierter teilweiser perspektivischer Darstellung,
Figur 2 einen Werkzeugteil des Instruments der Anordnung nach Figur 1, in perspektivischer teilweiser geschnittener Darstellung,
Figur 3 den Werkzeugteil nach Figur 2, in vertikal geschnittener Darstellung ohne Gewebe,
Figur 4 eine Energieversorgungsanordnung für das Instrument bzw. dessen Werkzeugteil nach den Figuren 2 und 3, in vereinfachter Schaltplandarstellung,
Figur 5 eine abgewandelte Ausführungsform der Energieversorgungsanordnung als vereinfachter Schaltplan und
Figur 6 das Instrument nah Figur 3 bei Koagulieren und Durchtrennen von biologischem Gewebe, in vertikal geschnittener Darstellung.

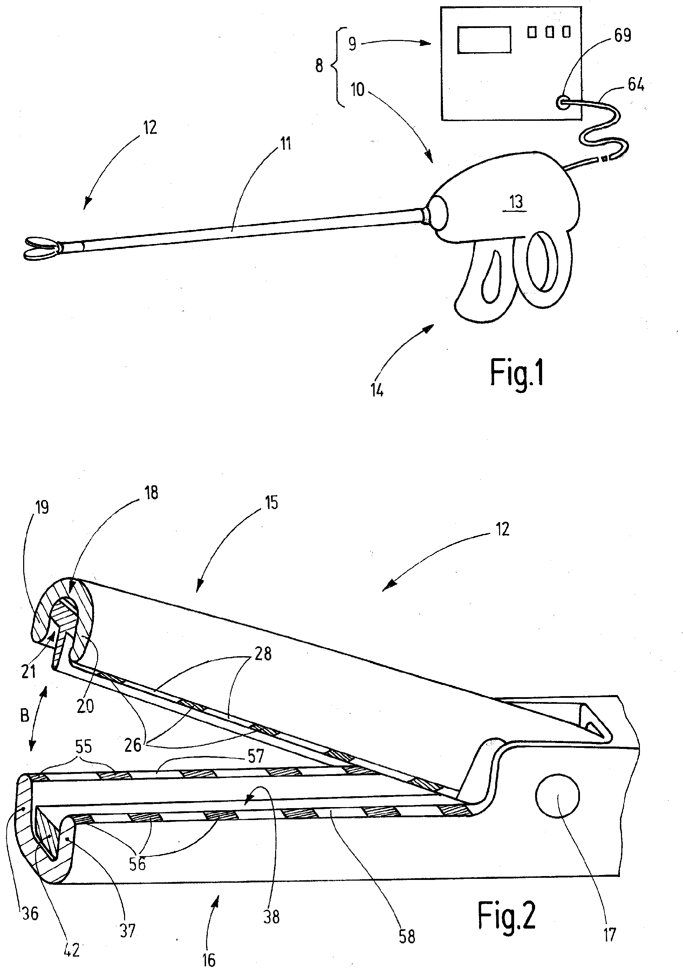

In Figur 1 ist ein System 8 veranschaulicht, zu dem ein Generator 9 und ein von diesem gespeistes chirurgisches Instrument 10 gehören. Der Generator 9 speist das Instrument 10 mit HF-Spannung, die zu einem an einem Schaft 11 gehaltenen Werkzeug 12 geleitet wird. Das proximale Ende des Schafts 11 ist mit einem Gehäuse 13 verbunden, an dem Betätigungselemente 14 zur Bewegung und Betätigung des Werkzeugs 12 angeordnet sind. Das Werkzeug 12 dient zum Durchtrennen von Gewebe unter Versiegelung der bleibenden Gewebesäume, so dass im Gewebe enthaltene Gefäße und Lumina an dem entstehenden Gewebesaum versiegelt sind.

Das in Figur 2 dargestellte Werkzeug 12 weist zwei Branchen 15, 16 auf, von denen mindestens eine um eine Schwenkachse 17 schwenkbar gelagert ist. Die Branchen 15, 16 sind durch Betätigung des Betätigungselements 14 aufeinander zu und durch Freigabe desselben voneinander weg bewegbar. Die Branchen 15, 16 können auch durch andere Mittel, beispielsweise pneumatische, hydraulische oder elektrische Antriebe, bewegt werden (nicht dargestellt).

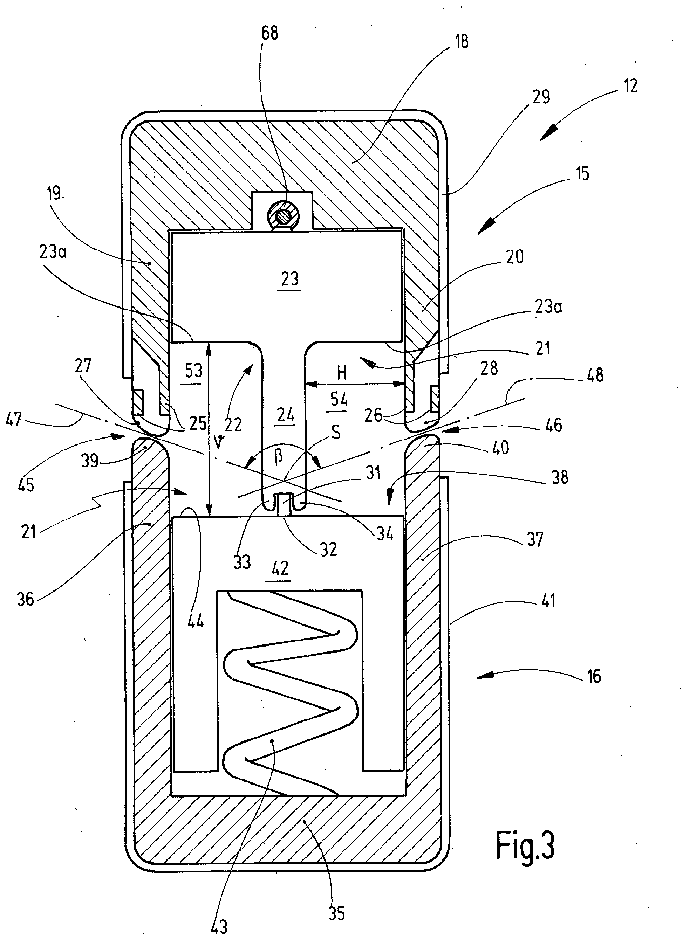

Die erste, in Figur 3 obere Branche 15, kann aus Metall, Keramik oder dergleichen bestehen. Ihr Grundkörper 18 ist vorzugsweise im Querschnitt U-profilförmig und weist zwei zueinander parallele erste Versiegelungselektroden 19, 20 auf, die elektrisch vorzugsweise miteinander verbunden sind und zwischen einander einen Raum 21 begrenzen. Dieser erstreckt sich über einen erheblichen Teil der Länge der ersten Branche 15 und dient der Aufnahme eines Schneidelektrodenträgers 22, der mit einem Fuß 23 von dem Raum 21 vorzugsweise unbeweglich aufgenommen ist. Von der der zweiten unteren Branche 16 zugewandten Fußfläche 23a erstreckt sich ein schmaler messerartiger Fortsatz 24 weg, der vorzugsweise aus dem Raum 21 herausragt, der vorzugsweise unterhalb einer die unteren Enden der Versiegelungselektroden 19, 20 miteinander verbindenden gedachten Linie endet.

Die Versiegelungselektrode 19, 20 ist vorzugsweise in Längsrichtung unterbrochen ausgebildet, so dass sie jeweils eine Reihe von Einzelelektroden 25, 26 aufweist, die elektrisch leitend mit dem Grundkörper 18 verbunden sein können. Die Einzelelektroden sind durch isolierende Bereiche 27, 28 voneinander getrennt. Diese können in einer Art Beschichtung auf die Versiegelungselektroden 19, 20 aufgetragen oder als Isolierkörper in den Grundkörper 18 eingebracht sein. Außerdem ist die Branche 15 vorzugsweise mit einem isolierenden Überzug 29 versehen, so dass der Grundkörper 18 nur an den Versiegelungselektroden 19, 20 nicht aber an sonstigen Stellen mit biologischem Gewebe in elektrischen Kontakt kommen kann.

Der Schneidelektrodenträger 22 weist an seiner Stirnseite eine Schneidelektrode 31 auf. Diese sitzt vorzugsweise in einer Nut oder Ausnehmung der unteren Schmalseite des messerartigen Fortsatzes 24, wobei die Schneidelektrode 31 mit einer Stirnfläche 32 frei liegt. Die Schneidelektrode 31 ist zwischen zwei Nutwänden 33, 34 gefasst, deren Breite vorzugsweise etwa so groß ist, wie die Breite der Schneidelektrode 31. Die Breite der Schneidelektrode 31 kann im Bereich von 0,05 mm bis 0,25 mm liegen und beträgt vorzugsweise 0,1 mm. Die Nutwände 33, 34 haben vorzugsweise eine Dicke von zum Beispiel 0,15 mm. Außerdem kann die Schneidelektrode 31 einen geringen Überstand über den Nutwänden bzw. Seitenwänden 33, 34 aufweisen. Der lediglich einige Mikrometer, beispielsweise 500 µm vorzugsweise 300 µm besonders vorzugsweise 200µm und bei besonderer Ausgestaltung vorzugsweise 0 µm bis 40 µm beträgt.

Der Fortsatz 24 überragt vorzugsweise die Koagulationselektroden 19, 20, so dass eine gedachte parallel zur Fußfläche 23a, die Koagulationselektroden 19, 20 verbindende, Linie den Fortsatz 24 vorzugsweise etwa auf halber Höhe schneidet.

Die zweite, in Figur 3 untere Branche 16, weist einen im Querschnitt vorzugsweise U-profilförmigen Grundkörper 35 aus vorzugsweise elektrisch leitendem Material auf. Seine beiden seitlichen Schenkel 36, 37 schließen zwischen einander einen Raum 38 ein und bilden mit ihren oberen Bereichen elektrisch leitende Gegenelektroden 39, 40 für die Versiegelungselektroden 19, 20 sowie die Schneidelektrode 31.

Der Grundkörper 35 ist an seiner Außenseite vorzugsweise mit einem isolierenden Überzug 41 versehen, der eine elektrischen Kontakt zu umgebenden biologischen Gewebe verhindert.

In dem Raum 38 ist ein vorzugsweise beweglich gelagertes Widerlagerelement 42 angeordnet, das beispielsweise über eine aus einer oder mehreren Federn bestehende Federanordnung 43 parallel zu den Schenkeln 36, 37 beweglich federn gehalten ist. Das Widerlagerelement 42 ist z.B. ein starres Keramikteil. Es kann aber auch aus einem nachgiebigen, insbesondere einem federnden Material, z.B. Elastomer ausgebildet sein. Es bildet dann selbst eine Federanordnung. Der Hub der wie auch immer ausgebildeten Federanordnung 43 ist dabei so bemessen, dass beim Schließen der Branchen und damit bei dem Aufeinanderfahren der Koagulationselektroden 19, 20 auf die Gegenelektroden 39, 40 ein Quetschspalt 45, 46 von Null möglich ist und damit der maximale Federweg noch nicht ausgenutzt ist. Das Widerlagerelement 42 befindet sich in Schließzustand in einer bevorzugten Ausführungsform etwas unterhalb der Gegenelektroden 39, 40.

Die der Schneidelektrode 31 zugewandten Seite des Widerlagerelements 42 ist vorzugsweise als ebene Druckfläche 44 ausgebildet. Bezüglich der Längsrichtung der Schneidelektrode 31 ist die Druckfläche 44 vorzugsweise entsprechend der Form der Schneidelektrode 31 ausgebildet, so dass diese spaltfrei an der Druckfläche 44 anliegen kann. Die Druckfläche 44 erstreckt sich vorzugsweise zwischen den Gegenelektroden 39, 40 und liegt bei komplett geschlossenen Branchen (ohne Gewebe) an der Stirnfläche 32 der Schneidelektrode 31 an.

Die Gestaltung der Druckfläche 44 kann durch die Materialwahl des Widerlagerelements 42 oder durch anwendungsbedingte Gegebenheiten anders gebildet sein. So kann beispielsweise die Druckfläche 44, wenn das Widerlagerelement 42 aus Elastomeren gebildet ist, nur im Bereich der Schneideelektrode 31 entsprechend der Form der Stirnseite 32 der Schneideelektrode 31 ausgebildet sein. Außerhalb dieses Bereichs kann die Druckfläche 44 bei komplett bzw. nahezu geschlossenen Branchen (ohne Gewebe) bezüglich der Stirnseite 32 der Schneidelektrode 31 ausgebildet (nicht dargestellt) erhaben oder vertieft ausgebildet sein.

Die Versiegelungselektroden 19, 20 und die Gegenelektroden 39, 40 definieren miteinander Quetschspalte 45, 46, die vorzugsweise, wie die Linien 47, 48 in Figur 3 andeuten, zu dem Widerlagerelement 42 abfallend ausgebildet sind, so dass die Linien 47, 48 miteinander einen stumpfen Winkel β einschließen und sich bei geschlossenem Werkzeug 12 oberhalb der Druckfläche 44 in einem Schnittpunkt S schneiden.

Zur funktionsbestimmenden Geometrie des Werkzeugs 12 gehören zwei Gewebeaufnahmeräume 53, 54. Diese sind zu beiden Seiten des Fortsatzes 24 ausgebildet. Sie werden vertikal zwischen dem Fuß 23 und der Druckfläche 44 begrenzt. Die Vertikalausdehnung V liegt bei geschlossenen Branchen zum Beispiel im Bereich von 0,7 mm bis zu 2,5 mm, vorzugsweise 1,4 mm. Die beiden Gewebeaufnahmeräume 53, 54 sind vorzugsweise gleich groß und weisen eine Horizontalausdehnung H auf, die durch den Abstand zwischen dem Fortsatz 24 und dem jeweiligen Schenkel 36, 37 definiert ist. Die Horizontalausdehnung H ist vorzugsweise deutlich größer als die Dicke der Schneidelektrode 31 wie auch größer als die Dicke des Fortsatzes 24 und/oder der Schenkel 36, 37. Vorzugsweise beträgt die Horizontalausdehnung H etwa das 0,2- bis 0,6-fache der Vertikalausdehnung V.

Die Gegenelektroden 39, 40 können an den Schenkeln 36, 37 längs durchgehend ausgebildet sein. Bevorzugt können sie jedoch auch, wie in Figur 2 angedeutet, entsprechend der Ausbildung der Versiegelungselektroden 19, 20 aus Einzelelektroden 55, 56 bestehen, die voneinander durch isolierende Bereiche 57, 58 getrennt sind. Vorzugsweise sind die isolierenden Bereiche 57, 58 in Branchenlängsrichtung länger als die Einzelelektroden 55, 56. Die isolierenden Bereiche 57, 58 können durch eine isolierende Beschichtung oder Isolierkörper gebildet sein. Außerdem sind die Einzelelektroden 55, 56 so gegen die Einzelelektroden 25, 26 versetzt, dass sie sich auch dann nicht berühren können, wenn der Quetschspalt 45, 46 Null ist und somit eine Berührung der Branchen 15, 16 eintritt. Jede Einzelelektrode 25, 26 trifft dann jeweils auf einen isolierenden Bereich 57, 58. Alternativ kann der Aufbau invers gestaltet sein. Die Versiegelungselektroden 19, 20 und die Gegenelektroden 39 40 können aus isolierendem Material mit elektrisch leitenden Einzelelektroden 55,56 gebildet sein.

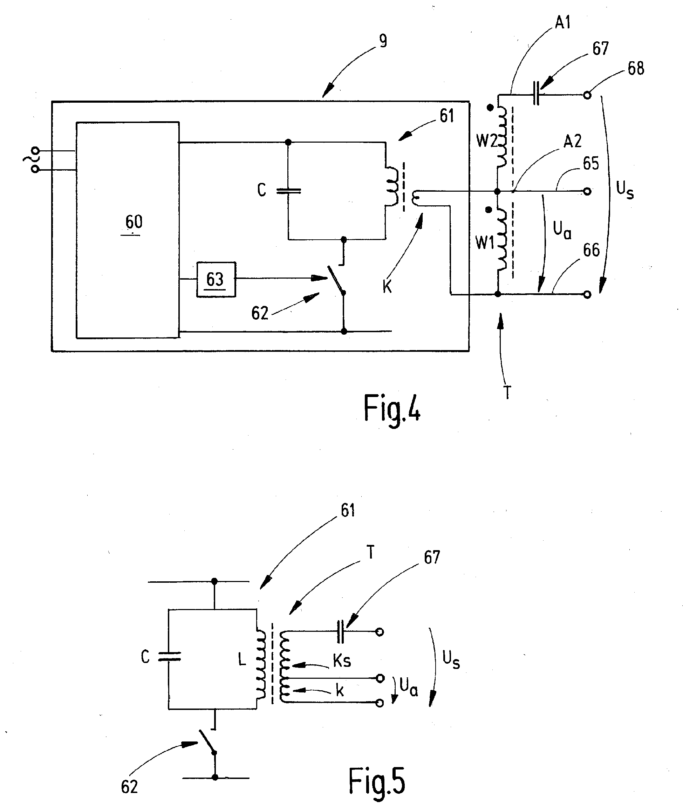

Der Generator 9 ist in Figur 4 schematisiert dargestellt. Er umfasst ein Netzteil 60, das eine Gleichspannung für einen HF-Generator 61 bereitstellt. Dieser besteht aus einem Schwingkreis mit einem Kondensator C und einer Spule L und wird von einem gesteuerten Verstärker- oder Schaltelement 62 abhängig von den Vorgaben eines Steuermoduls 63 erregt.

An der Spule L wird über eine Koppelwicklung K HF-Leistung ausgekoppelt, um diese über eine Leitung 64 (Figur 1) dem Instrument 10 zuzuführen. Die HF-Leistung dient zur Bestromung sowohl der Versiegelungselektroden 19, 20 als auch der Schneidelektrode 31. Zur Aufsplittung der Leistung dient ein Transformator T mit einer Primärwicklung W1 und mindestens einer Sekundwicklung W2. Vorzugsweise ist der Transformator T als Spartransformator ausgebildet. Die Primärwicklung W1 ist mit dem Ausgang des HF-Generators 61, d.h. beispielsweise mit seiner Koppelspule (Koppelwicklung) K verbunden. Ist der Transformator T ein Spartransformator, ist sein Eingang (d.h. das obere Ende seiner Primärwicklung) zugleich sein Ausgang A2. Von diesem Ausgang A2 und somit dem Ausgang des HF-Generators 61 führt eine Leitung 65 zu den Versiegelungselektroden 19, 20.

Eine Masseleitung 66, die mit dem unteren Ende der Wicklung W1 und der Koppelwicklung K verbunden ist, führt zu den Gegenelektroden 39, 40. Damit ist zwischen der Versiegelungselektrode 19 und/oder 20 und der Gegenelektrode 39 und/oder 40 gefasstes biologisches Gewebe parallel zu der Wicklung W1 geschaltet und direkt an den Ausgang des HF-Generators 61 angeschlossen. Das Gewebe wird mit der Ausgangsspannung Uₐ zur Versiegelung beaufschlagt.

Zur Bereitstellung eine Schneidspannung Uₛ ist die Wicklung W2 mit ihrem unteren Ende mit der Leitung 65 und mit ihrem oberen Ende über ein Strombegrenzungselement 67 vorzugsweise über einen Koppelkondensator 67 und eine Leitung 68 mit der Schneidelektrode 31 verbunden. Die Sekundärwicklung W2 ist gleichsinnig zu der Primärwicklung W1 gepolt, so dass die Schneidspannung Uₛ die Summe aus der Ausgangsspannung Uₐ und der von der Wicklung W2 abgegebenen Spannung ist, wobei diese Summe größer als die Ausgangsspannung Uₐ ist. Der Transformator T hat eine geringe Streuinduktivität und einen geringen Innenwiderstand. Der Koppelkondensator 67 wirkt strombegrenzend und führt dazu, dass die Versiegelungselektrode(n) 19, 20 mit geringem Innenwiderstand und die Schneidelektrode 31 mit erhöhtem Innenwiderstand gespeist werden.

Der Transformator T kann in den Generator 9 oder alternativ in das Instrument 10 eingebaut sein. Weiter alternativ kann er in die Leitung 64 oder den daran vorgesehenen Stecker 69 oder ein nicht dargestelltes Zwischensteckmodul eingebaut sein. Wie Figur 5 zeigt, ist es auch möglich, den Transformator T und die Spule L des HF-Generators 61 zu vereinigen. Zusätzlich zu der Koppelwicklung K ist dann eine zweite Koppelwicklung KS für die Schneidspannung Uₛ vorgesehen, die wiederum über den Koppelkondensator 67 die HF-Spannung nach außen gibt.

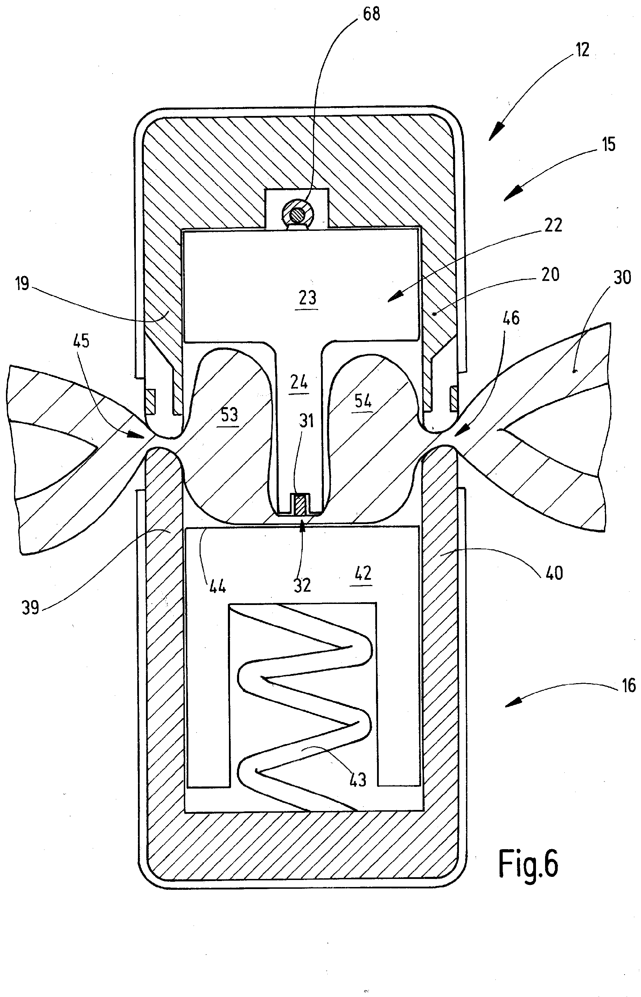

Das insoweit beschriebene System 8 arbeitet wie folgt:
Zur Versiegelung von Hohlgefäßen, zum Trennen von Körpergeweben, insbesondere Geweben, die mit Blutgefäßen durchsetzt sind und somit an den Trennsäumen zu versiegeln sind, wird solches Gewebe 30, wie Figur 6 veranschaulicht, mit dem Werkzeug 12 zwischen den Branchen 15, 16 gefasst, wobei die Branchen aufeinander zu bewegt werden. Sobald das Werkzeug 12 ausreichend geschlossen ist, wird der HF-Generator 61 aktiviert, so dass gleichzeitig sowohl die Versiegelungselektroden 19, 20 als auch die Schneidelektrode 31 gegenüber den Gegenelektroden 39, 40 unter Spannung gesetzt und somit das Gewebe bestromt wird. Das Gewebe wird in den Quetschspalten 45, 46 zusammengedrückt und durch den von den Versiegelungselektroden 19, 20 zu den Gegenelektroden 39, 40 fließenden Strom erwärmt, denaturiert und versiegelt. In den Gewebeaufnahmeräumen 53, 54 schrumpft das Gewebe weniger oder nicht. Von der gleichzeitig unter Spannung gesetzten Schneidelektrode 31 ausgehender Strom hat an der Stirnfläche 32 eine hohe Stromdichte, so dass das Gewebe dort durch schnelle Austrocknung und durch die hohe Spannung an der Schneidelektrode 31 auftretende Schnittfunken durchtrennt wird. Die Stromdichte in den Gewebeaufnahmeräumen 53, 54 ist jedoch gering, so dass hier kaum eine Gewebeschrumpfung auftritt. Die sich bildenden Gewebewulste verhindern, selbst wenn das Gewebe vor Abschluss des Versiegelungsprozesses durchtrennt ist, ein Herausrutschen der Gewebesäume aus dem Werkzeug 12.
Der Schneidstrom wird durch den Koppelkondensator 67 begrenzt. Die Schneidspannung ist so hoch, dass nach Austrocknung und Denaturierung des Gewebes 45 durch den Schneidstrom an der Schneidelektrode 31 Schnittfunken entstehen können, um eine Gewebedurchtrennung zu bewirken. Der Koppelkondensator 67 ist jedoch so bemessen, dass ein Stromanstieg auf Maße, die zu hohen Stromdichten in den Gewebeaufnahmeräumen führen würden, sicher ausgeschlossen werden. Damit wird verhindert, dass in den Gewebeaufnahmeräumen 53, 54 sitzendes Gewebe übermäßig schrumpft und dadurch durch die Quetschspalte 45, 46 entkommt, bevor der Versiegelungsprozess beendet ist.

Das Zusammenspiel von hoher Schneidspannung Uₛ und Koppelkondensator 67 begrenzt den Schneidstrom während der Austrocknungsphase des Gewebes im Bereich der Schneidelektrode und stellt in Verbindung mit der dargestellten Werkzeuggeometrie den Schlüssel zum zügigen und zuverlässigen Durchtrennen von biologischem Gewebe mit hoher Schnittqualität und hoher Prozesssicherheit sicher.

Zur Strombeeinflussung kann alternativ oder zusätzlich ein Koppelkondensator in der Leitung 65 vorgesehen sein. Außerdem kann anstelle des Koppelkondensators 67 ein anderes strombegrenzendes Bauelement oder eine Zusammenschaltung von Bauelementen vorgesehen werden, die einen oder mehrere Kondensatoren enthalten kann.

Ein zum gleichzeitigen Koagulieren und Durchtrennen von Gewebe vorgesehenes Instrument 10 weist zwischen Schneidelektrode 31 und Versiegelungselektrode 19 einen Gewebeaufnahmeraum 53 zur Ausbildung eines Gewebewulstes zur Sicherung des Gewebes im Werkzeug 12 während des Versiegelungsprozesses auf. Um die Ausbildung voluminöser Gewebewulste zu ermöglichen und deren Schrumpfung zu verhindern, wird die Schneidelektrode 31 über ein Strombegrenzungsbauelement vorzugsweise in Gestalt eines Koppelkondensators 67 gespeist. Damit wird insbesondere bei Geweben, die leicht zu durchtrennen sind, jedoch eine lange Versiegelungszeit benötigen, eine hohe Prozesssicherheit erreicht.

**Bezugszeichen:**

| | |
|---|---|
| 8 | System |
| 9 | Generator |
| 10 | Instrument |
| 11 | Schaft |
| 12 | Werkzeug |
| 13 | Gehäuse |
| 14 | Betätigungselement |
| 15, 16 | Branchen |
| 17 | Schwenkachse |
| 18 | Grundkörper |
| 19, 20 | erste (obere) Versiegelungs- Koagulationselektroden |
| 21 | Raum zwischen den Versiegelungselektroden 19, 20 |
| 22 | Schneidelektrodenträger |
| 23 | Fuß des Schneidelektrodenträgers |
| 23a | Fußfläche |
| 24 | Fortsatz |
| 25, 26 | Einzelelektroden der Versiegelungselektroden 19, 20 |
| 27, 28 | isolierende Bereiche |
| 29 | isolierender Überzug |
| 30 | Gewebe |
| 31 | Schneidelektrode |
| 32 | Stirnfläche |
| 33, 34 | Nutwände |
| 35 | Grundkörper |
| 36, 37 | Schenkel des Grundkörpers 35 |
| 38 | Raum |
| 39, 40 | Gegenelektroden |
| 41 | isolierender Überzug |
| 42 | Widerlagerelement |
| 43 | Federelement |
| 44 | Druckfläche |
| 45, 46 | Quetschspalte |
| 47, 48 | Linien |
| β | stumpfer Winkel |
| S | Schnittpunkt |
| 53, 54 | Gewebeaufnahmeräume |
| v | Vertikalausdehnung der Gewebeaufnahmeräume 53, 54 |
| H | Horizontalausdehnung der Gewebeaufnahmeräume 53, 54 |
| 55, 56 | Einzelelektroden |
| 57, 58 | isolierende Bereiche |
| 59 | |
| 60 | Netzteil |
| 61 | HF-Generator |
| C | Kondensator |
| L | Spule |
| K | Koppelwicklung |
| 62 | Schaltelement |
| 63 | Steuermodul |
| T | Transformator |
| 64 | Leitung |
| 65 | Leitung |
| 66 | Masseleitung |
| Uₐ | Ausgangsspannung |
| 67 | Strombegrenzungselement, Koppelkondensator |
| Uₛ | Schneidspannung |
| 68 | Leitung |
| 69 | Stecker |
| KS | Koppelwicklung |
| | |
| | |

## Patentansprüche

1. System (8):
mit einem Instrument (10), das
mindestens eine Koagulationselektrode (19),
mindestens eine Schneidelektrode (31) sowie mindestens eine Gegenelektrode (39) aufweist,
wobei die Schneidelektrode (31), Koagulationselektrode (19) und die Gegenelektrode (39) an Branchen (15, 16) eines Werkzeugs (12) angeordnet sind, von denen eine auf die andere hin und von dieser weg bewegbar ist,
und
mit einer Energieversorgungsanordnung,
bestehend aus einem Generator (9) und einem Transformator (T), der
einen ersten Ausgang (A1), der mit der Schneidelektrode (31) verbunden ist,
und einen zweiten Ausgang (A2) aufweist, der mit der Koagulationselektrode (19) verbunden ist,
wobei wenigstens ein Strombegrenzungsbauelement (67) zwischen dem ersten Ausgang (A1) und der Schneidelektrode (31) und/ oder zwischen dem zweiten Ausgang (A2) und der Koagulationselektrode (19) angeordnet ist,
wobei die Schneidelektrode (31) eine elektrisch leitende Stirnfläche (32) aufweist und an Seitenflächen weitestgehend elektrisch isolierend ausgebildet ist,
die der Koagulationselektrode (19) zugewandt sind, **dadurch gekennzeichnet, dass** die Energieversorgungsanordnung so ausgelegt ist,
dass das Schneiden des Gewebes beendet ist, bevor der Versiegelungsprozess des Gewebes beendet ist,
dass die Schneidelektrode (31) und die Gegenelektrode (39) an zwei unterschiedlichen Branchen (15, 16) des Instruments (10) angeordnet sind, von denen wenigstens eine Branche (15) auf die andere zu und von dieser weg beweglich ist und die einen Quetschspalt (45, 46) festlegen, der von der Schneidelektrode (31) durch einen Gewebeaufnahmeraum (53, 54) getrennt ist, und
dass der Gewebeaufnahmeraum (53, 54) sich sowohl oberhalb des Quetschspalts (45, 46) in eine der Branchen (15, 16) als auch unterhalb des Quetschspalts (45, 46) in die andere der Branchen (15, 16) hinein erstreckt.

2. System nach einem der vorigen Ansprüche, **dadurch gekennzeichnet, dass** der Schneidelektrode (31) gegenüberliegend ein isolierendes Widerlagerelement (42) angeordnet ist.

3. System nach Anspruch 2, **dadurch gekennzeichnet, dass** das Widerlagerelement (42) beweglich gelagert ist.

4. System nach Anspruch 3, **dadurch gekennzeichnet, dass** das Widerlagerelement (42) federnd gelagert oder ausgebildet ist.

5. System nach einem der vorigen Ansprüche, **dadurch gekennzeichnet, dass** die Koagulationselektrode (19, 20) durch eine Reihe voneinander beabstandeter Einzelelektroden (25, 26) gebildet ist.

6. System nach einem der vorigen Ansprüche, **dadurch gekennzeichnet, dass** die Gegenelektrode (39, 40) durch eine Reihe voneinander beabstandeter Einzelelektroden (55) und (56) gebildet ist.

7. System nach einem der vorigen Ansprüche, **dadurch gekennzeichnet, dass** die Einzelelektroden (19, 20) der Koagulationselektrode (19) und die Einzelelektroden (55, 56) der Gegenelektrode (39, 40) einander nicht überlappend angeordnet sind.

8. System nach einem der vorigen Ansprüche, **dadurch gekennzeichnet, dass** der Transformator (T) derart ausgebildet ist, dass der erste Ausgang (A1) eine höhere Spannung liefert als der zweite Ausgang (A2).

9. System nach einem der vorigen Ansprüche, **dadurch gekennzeichnet, dass** die Impedanz des Strombegrenzungsbauelements (67) größer ist als der Innenwiderstand des Transformators (T) an seinem ersten Ausgang (A1).

10. System nach einem der vorigen Ansprüche, **dadurch gekennzeichnet, dass** die Impedanz des Strombegrenzungsbauelements (67) größer ist als der Innenwiderstand des Transformators (T) an seinem zweiten Ausgang (A2).

11. System nach einem der vorigen Ansprüche, **dadurch gekennzeichnet, dass** der Transformator (T) eine Wicklung (W1) aufweist, die mit einer Koagulationsspannung (Uₐ) versorgt ist und dass vorzugsweise die an den Ausgängen (A1) und (A2) bereitgestellten Ausgangsspannungen so bemessen sind, dass das Schneiden zeitgleich mit dem Koagulieren beginnt und vor dem Ende des Koagulationsvorgangs beendet ist.

12. System nach Anspruch 1, 3 oder 4, **dadurch gekennzeichnet, dass** das Widerlagerelement (42) aus einem Elastomer ausgebildet ist.

## Claims

1. A system (8):
with an instrument (10) comprising
at least one coagulation electrode (19),
at least one cutting electrode (31), and
at least one counter electrode (39),
wherein the cutting electrode (31), the coagulation electrode (19) and the counter electrode (39) are arranged on branches (15, 16) of a tool (12), one of which is movable toward and away from the other, and
with an energy supply arrangement
consisting of a generator (9) and a transformer (T) which has a first output (A1) connected to the cutting electrode (31)
and a second output (A2) connected to the coagulation electrode (19),
wherein at least one current-limiting component (67) is arranged between the first output (A1) and the cutting electrode (31) and/or between the second output (A2) and the coagulation electrode (19),
wherein the cutting electrode (31) has an electrically conductive end face (32) and is configured to be largely electrically insulating on side faces facing the coagulation electrode (19)
**characterized in that** the energy supply arrangement is designed such that the cutting of the tissue is completed before the tissue sealing process is completed,
that the cutting electrode (31) and the counter electrode (39) are arranged on two different branches (15, 16) of the instrument (10), of which at least one branch (15) is movable toward and away from the other and which define a crushing gap (45, 46) separated from the cutting
electrode (31) by a tissue receiving space (53, 54), and that the tissue receiving space (53, 54) extends both above the crushing gap (45, 46) into one of the branches (15, 16) and below the crushing gap (45, 46) into the other of the branches (15, 16).

2. The system according to any of the preceding claims, **characterized in that** an insulating abutment element (42) is arranged opposite the cutting electrode (31).

3. The system according to claim 2, **characterized in that** the abutment element (42) is movably mounted.

4. The system according to claim 3, **characterized in that** the abutment element (42) is resiliently mounted or designed.

5. The system according to any of the preceding claims, **characterized in that** the coagulation electrode (19, 20) is formed by a series of spaced-apart individual electrodes (25, 26).

6. The system according to any of the preceding claims, **characterized in that** the counter electrode (39, 40) is formed by a series of spaced-apart individual electrodes (55) and (56).

7. The system according to any of the preceding claims, **characterized in that** the individual electrodes (19, 20) of the coagulation electrode (19) and the individual electrodes (55, 56) of the counter electrode (39, 40) are arranged so as not to overlap one another.

8. The system according to any of the preceding claims, **characterized in that** the transformer (T) is configured such that the first output (A1) supplies a higher voltage than the second output (A2).

9. The system according to any of the preceding claims, **characterized in that** the impedance of the current-limiting component (67) is greater than the internal resistance of the transformer (T) at its first output (A1).

10. The system according to any of the preceding claims, **characterized in that** the impedance of the current-limiting component (67) is greater than the internal resistance of the transformer (T) at its second output (A2).

11. The system according to any of the preceding claims, **characterized in that** the transformer (T) has a winding (W1) which is supplied with a coagulation voltage (Uₐ), and that preferably the output voltages provided at the outputs (A1) and (A2) are dimensioned such that the cutting begins at the same time as the coagulation and is completed before the end of the coagulation process.

12. The system according to claim 1, 3 or 4, **characterized in that** the abutment element (42) is formed from an elastomer.

## Revendications

1. Système (8) :
comprenant un instrument (10) qui présente
au moins une électrode de coagulation (19),
au moins une électrode de coupe (31), ainsi que
au moins une contre-électrode (39),
dans lequel l'électrode de coupe (31), l'électrode de coagulation (19) et la contre-électrode (39) sont disposées sur des mors (15, 16) d'un outil (12), dont l'un peut être déplacé en direction de l'autre et en être éloigné,
et
comprenant un ensemble d'alimentation en énergie constitué d'un générateur (9) et d'un transformateur (T) qui présente une première sortie (A1), reliée à l'électrode de coupe (31), et une deuxième sortie (A2) qui est reliée à l'électrode de coagulation (19),
au moins un composant limiteur de courant (67) étant disposé entre la première sortie (A1) et l'électrode de coupe (31) et/ou entre la deuxième sortie (A2) et l'électrode de coagulation (19), l'électrode de coupe (31) présentant une face frontale (32) électriquement conductrice et étant réalisée en majeure partie de façon électriquement isolante sur les faces latérales qui sont tournées vers l'électrode de coagulation (19),
**caractérisé en ce que**
l'ensemble d'alimentation en énergie est conçu pour que la coupe des tissus soit terminée avant que le processus de scellement des tissus ne soit terminé,
**en ce que** l'électrode de coupe (31) et la contre-électrode (39) sont disposées sur deux mors (15, 16) différents de l'instrument (10), dont au moins un mors (15) peut être déplacé en direction de l'autre et en être éloigné, et qui définissent entre eux une fente de pincement (45, 46) qui est séparée de l'électrode de coupe (31) par un espace accueillant les tissus (53, 54), et
**en ce que** l'espace accueillant les tissus (53, 54) s'étend à la fois dans l'un des mors (15, 16), au-dessus de la fente de pincement (45, 46), et dans l'autre mors (15, 16), en dessous de la fente de pincement (45, 46).

2. Système selon une des revendications précédentes, **caractérisé en ce qu'**un élément de butée (42) isolant est disposé en vis-à-vis de l'électrode de coupe (31).

3. Système selon la revendication 2, **caractérisé en ce que** l'élément de butée (42) est monté avec possibilité de déplacement.

4. Système selon la revendication 3, **caractérisé en ce que** l'élément de butée (42) est monté ou réalisé de manière élastique.

5. Système selon une des revendications précédentes, **caractérisé en ce que** l'électrode de coagulation (19, 20) est constituée d'une série d'électrodes individuelles (25, 26) espacées les unes des autres.

6. Système selon une des revendications précédentes, **caractérisé en ce que** la contre-électrode (39, 40) est constituée d'une série d'électrodes individuelles (55) et (56) espacées les unes des autres.

7. Système selon une des revendications précédentes, **caractérisé en ce que** les électrodes individuelles (19, 20) de l'électrode de coagulation (19) et les électrodes individuelles (55, 56) de la contre-électrode (39) sont disposées de manière à ne pas se chevaucher.

8. Système selon une des revendications précédentes, **caractérisé en ce que** le transformateur (T) est réalisé de manière à ce que la première sortie (A1) fournisse une tension plus élevée que la deuxième sortie (A2).

9. Système selon une des revendications précédentes, **caractérisé en ce que** l'impédance du composant limiteur de courant (67) est plus grande que la résistance interne du transformateur (T) à sa première sortie (A1).

10. Système selon une des revendications précédentes, **caractérisé en ce que** l'impédance du composant limiteur de courant (67) est plus grande que la résistance interne du transformateur (T) à sa deuxième sortie (A2).

11. Système selon une des revendications précédentes, **caractérisé en ce que** le transformateur (T) présente un enroulement (W1) qui est alimenté avec une tension de coagulation (Uₐ), et **en ce que** les tensions de sortie délivrées aux sorties (A1) et (A2) sont de préférence prévues telles que la coupe commence en même temps que la coagulation et soit terminée avant la fin du processus de coagulation.

12. Système selon la revendication 1, 3 ou 4, **caractérisé en ce que** l'élément de butée (42) est réalisé à partir d'un élastomère.
